# EUROPEAN PATENT APPLICATION

(11) **EP 1 201 237 A1**
(43) Date of publication of application: **02.05.2002**
(21) Application number: 00309566.8
(22) Date of filing: 30.10.2000
(51) Int. Cl.: A61K 31/16, A61K 31/4453, A61K 31/5375, A61K 31/10, A61P 35/00, A61P 19/02, A61P 37/02, A61P 3/10, A61P 31/12, A61P 43/00

(54) **Composition comprising an amide or a dialkylsulphoxide derivative for the treatment of cancer**

(71) Applicant: Virodene Pharmaceutical Holdings (PTY) Ltd., Rietfontein 0084 (ZA)
(72) Inventor: Visser, Michelle Olga Patricia Giesteira, Rietfontein, 0084 (ZA)
(74) Representative: Spall, Christopher John

(57) **Abstract**

A substance or composition for the treatment of cancer, rheumatoid arthritis, wasting syndrome diseases, immunomodulatory related diseases, sugar diabetes mellitus 1 or 2 or hepatitis A, B or C comprises an active agent selected from the group consisting of amides of the general formula R₃-CO-NR₁,R₂, in which:
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)ₙ, wherein n = 4 or 5, or are (CH₂) ₂O (CH₂)₂; and
R₃ is selected from H, Me and saturated and unsaturated C₂ - C₃ alkyl groups;
dialkylsulphoxides of the general formula R₄R₅SO, and mixtures thereof.

## Description

The invention relates, in particular, to a substance or composition for the treatment of cancer, rheumatoid arthritis, wasting syndrome diseases, immuno modulatory related diseases, sugar diabetes mellitus 1 or 2 or hepatitis A, B or C, to a method of making a medicament or preparation for the treatment of cancer, rheumatoid arthritis, wasting syndrome diseases, immunomodulatory related diseases, sugar diabetes mellitus 1 or 2 or hepatitis A, B or C, to the use in the treatment of cancer, rheumatoid arthritis, wasting syndrome diseases, immuno-modulatory related diseases, sugar diabetes mellitus 1 or 2 or hepatitis A, B or C, of a medicament or preparation, to the use of a substance or composition in the manufacture of a medicament or preparation for the treatment of cancer, rheumatoid arthritis, wasting syndrome diseases, immunomodulatory related diseases, sugar diabetes mellitus 1 or 2 or hepatitis A, B or C to a method of treating cancer, rheumatoid arthritis, wasting syndrome diseases, immuno-modulatory related diseases, sugar diabetes mellitus 1 or 2 or hepatitis A, B or C and to a dosage form.

According to one aspect of the invention, there is provided a substance or composition for the treatment of cancer, rheumatoid arthritis, wasting syndrome diseases, immunomodulatory related diseases, sugar diabetes mellitus 1 or 2 or hepatitis A, B or C, the substance or composition comprising an active agent selected from the group consisting of amides of the general formula R₃-CO-NR₁,R₂, in which:
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5,
   or (CH₂)₂O(CH₂)₂; and
R₃ is selected from H, Me and saturated and unsaturated C₂ - C₃ alkyl groups;
dialkylsulphoxides of the general formula R₄R₅SO and mixtures thereof.

At least one of R₁ and R₂ may be a methyl group. At least one of R₄ and R₅ may be a methyl group.

Instead, at least one of R₁, R₂, R₄ and R₅ may be a fluorinated C₁-C₃ alkyl group.

The active agent may be selected from the group including formamide, methyl formamide, dimethylformamide, acetamide, methylacetamide, dimethylacetamide, diethylacetamide, isopropylacetamide, diisopropylacetamide, N-acetylpiperidine, N(β-hydroxyethyl) acetamide, N,N-di(β-hydroxyethyl) acetamide, N--acetylmorpholine, acrylamide, propionamide, N-fluoromethyl-N-methylformamide, dimethylsulphoxide and mixtures of any two or more thereof.

In one particular embodiment, the active agent may be dimethylformamide C₃H₇NO (DMF). In another particular embodiment the active agent may be HCON (CH₃) (CH₂F). In another particular embodiment, the active agent may be dimethylsulphoxide (DMSO).

DMF is generally used as a polar solvent and is readily absorbed through the skin, through the lungs or after oral exposure. The absorption rate of liquid DMF through the skin amounts to about 9.4 mg per cm² per hour. DMF is rapidly metabolized, the main biotransformation site is the liver and excretion occurs for the larger part via the urine. The main metabolites in rat, mice, hamster and man are N-(hydroxymethyl)-N-methylformamide (HMMF), N-(hydroxymethyl)-formamide (HMF) and N-acetyl-S-(N-methyl-carbamoyl) cysteine (AMCC). Unchanged DMF is excreted in the urine as a small fraction of the dose. The limited data available indicate that a significant amount of the dose remains unexcreted and/or is excreted as unidentified compounds.

DMF has low acute dermal, oral and inhalation toxicity. It is considered to be a mild to moderate skin and eye irritant and readily permeates the skin. There is no indication of skin sensitizing properties.

A NOAEL (No-Adverse-observed-Effect-Level) could not be established in several 90-day studies. In a 28 day inhalation study with dogs, no effects were found at 63 mg/m³. In another study with dogs, reversible cardiovascular effects were found at 6 0 mg/m³.

The NOAEL for developmental effects amounted to 44 mg/kg body weight in an oral study and 150 mg/m³ in an inhalation study with rabbits.

A draft interim clinical safety report produced by Guy's Drug Research Unit of Kings College and St Thomas' Hospitals' Medical and Dental School is set out below. The safety report shows that a dose of 7,06 g of DMF administered transdermally once a week for a period of 8 hours to human subjects is non-toxic, has no serious adverse events, and does not result in raised liver enzymes.

### DRAFT INTERIM CLINICAL SAFETY REPORT

A single dose study of the disposition, safety, tolerability and pharmacokinetics of virodene P058 in healthy volunteers.
PROTOCOL NUMBER: 98/VIRO/PO58/01
DOSE: 1 patch containing 7060mg DMF
SUBJECT NUMBERS: 001 to 010 inclusive

A single dose study of the disposition, safety, tolerability and pharmacokinetics of virodene P058 in healthy volunteers
Protocol number: 98/VIRO/P058/01
DRAFT INTERIM SAFETY REPORT

### GENERAL

° 5 subjects dosed on day 1 (subjects 001 - 005)
° 5 subjects dosed on day 14 (subjects 006 - 010)
° No withdrawals
° Final follow up for subjects 001 - 005 occurred on day 15
° Final follow up for subjects 006 - 010 due on day 28

**Table 1.1 -**

| Subject numbers and initials | | |
|---|---|---|
| Allocation Number | Subject Initials | Date Dosed |
| 001 | GDT | day 1 |
| 002 | JCG | day 1 |
| 003 | CGM | day 1 |
| 004 | JAB | day 1 |
| 005 | MBV | day 1 |
| 006 | C P | day 14 |
| 007 | CKN | day 14 |
| 008 | RAK | day 14 |
| 009 | GRG | day 14 |
| 010 | CPD | day 14 |

### DOSE

1 patch containing 7060mg of DMF was applied to the skin on the deltoid region of either shoulder. The first patch was applied at 10h00 on day 1 for subjects 001 - 005 and at 09h20 on day 1 for subjects 006 - 010 with 10 minute intervals between dosing.

### SAFETY ASSESSMENTS

° Laboratory Safety Values: No clinically significant changes observed for biochemistry, liver function tests, haematology or coagulation parameters.
° Vital Signs - There were no obvious clinically significant changes observed
° Continuous lead [I monitoring - No clinically significant changes observed]
° 12 kead ECG - No clinically significant changes observed
° Adverse events are reported in Table 1.2

### CLINICAL SAFETY OBSERVATIONS

° There were no serious adverse events
° The blind was not broken

*The data and statements contained in this report are based on clinical observations and have not been subject to any formal statistical analysis or Q.A. audit.*

**Table 1. 2**

| **A summary of adverse events reported and observed for subjects** | | | | | | | |
|---|---|---|---|---|---|---|---|
| Sub No | Description | Severity | Relationship | Time patch applied | Onset | Offset | Action taken |
| 001 | Dizziness | Mild | None | 10:00 Day 1 | Predose | Predose | None |
| 001 | Itchiness to patch site | Mild | Possible | 10:00 Day 1 | 10:04 Day 1 | 10:28 Day 1 | None |
| 001 | Hot feeling to patch site | Mild | Possible | 10:00 Day 1 | 10:20 Day 1 | 10:28 Day 1 | None |
| 001 | Stinging feeling to patch site | Mild | Possible | 10:00 Day 1 | 10:20 Day 1 | 10:00 Day 2 | None |
| 001 | Hot and sweaty all over | Mild | Possible | 10:00 Day 1 | 10:40 Day 1 | 11:25 Day 1 | None |
| 001 | Redness under tape | Mild | None | 10:00 Day 1 | 13:56 Day 1 | 10:46 Day 3 | None |
| 001 | Stinging to patch site | Mild | Possible | 10:00 Day 1 | 18:01 Day 1 | 12:00 Day 4 | None |
| 001 | Erythema and blistering to patch site | Mild | Possible | 10:00 Day 1 | 18:02 Day 1 | 10:05 Day 11 | None |
| 001 | Diarrhoea | Mild | Possible | 10:00 Day 1 | 13:00 Day 3 | 13:10 Day 3 | None |
| 001 | Tingling in hands | Mild | Remote | 10:00 Day 1 | 10:00 Day 4 | 10:06 Day 11 | None |
| 001 | Peeling skin in both hands | Mild | Remote | 10:00 Day 1 | 10:06 Day 4 | Ongoing | None |
| 001 | Lower back pain | Mild | None | 10:00 Day 1 | 13:00 Day 9 | 07:00 Day 12 | None |
| 002 | Erythema on plaster site | Mild | None | 10:10 Day 1 | 18:10 Day 1 | 20:05 Day 1 | None |
| 003 | Warm sensation under patch | Mild | Possible | 10:20 Day 1 | 11:30 Day 1 | 20:00 Day 1 | None |
| 003 | Erythema at patch site | Mild | Possible | 10:20 Day 1 | 18:21 Day 1 | 10:30 Day 6 | None |
| 003 | Sensitivity to patch site | Mild | Possible | 10:20 Day 1 | 18:21 Day 1 | 10:30 Day 6 | None |
| 003 | Dry skin to patch site | Mild | Possible | 10:20 Day 1 | 09:30 Day 2 | 10:00 Day 15 | None |
| 003 | Pruritis to patch site | Mild | Possible | 10:20 Day 1 | 00:00 Day 2 | 08:30 Day 3 | None |
| 004 | Cough | Mild | None | 10:30 Day 1 | Predose | 11:18 Day 3 | None |
| 004 | Shaving cut to face | Mild | None | 10:30 Day 1 | Predose | 22:30 Day 2 | None |
| 004 | Tingling to patch site | Mild | Possible | 10:30 Day 1 | 10:56 Day 1 | 13:15 Day 1 | None |
| 004 | Erythema to patch site | Mild | Possible | 10:30 Day 1 | 18:31 Day 1 | 11:07 Day 11 | None |
| 004 | Dry skin to patch site | Mild | Remote | 10:30 Day 1 | 11:07 Day 11 | Ongoing | None |
| 005 | Itchiness to patch site | Mild | Possible | 10:40 Day 1 | 10:49 Day 1 | 18:00 Day 1 | None |
| 005 | Redness under plaster | Mild | None | 10:40 Day 1 | 10:55 Day 1 | 22:41 Day 3 | None |
| 005 | Burning feeling to patch site | Mild | Possible | 10:40 Day 1 | 11:17 Day 1 | 13:30 Day 1 | None |
| 005 | Stinging feeling to patch site | Mild | Possible | 10:40 Day 1 | 11:17 Day 1 | 13:30 Day 1 | None |
| 005 | Erythema and blistering at patch site | Mild | Possible | 10:40 Day 1 | 18:41 Day 1 | 09:00 Day 16 | None |
| 005 | Erythema at electrode sites | Mild | Remote | 10:40 Day 1 | 10:45 Day 2 | 12:00 Day 4 | None |
| 005 | Itchiness at patch site | Mild | Possible | 10:40 Day 1 | 08:30 Day 2 | 08:30 Day 3 | None |
| 005 | Drowsiness | Mild | Remote | 10:40 Day 1 | 10:00 Day 2 | 08:30 Day 3 | None |
| 005 | Nausea | Mild | Remote | 10:40 Day 1 | 22:00 Day 2 | 08:00 Day 4 | None |
| 006 | Constricting feeling to right arm | Mild | None | 09:20 Day 1 | 09:48 Day 1 | 18:07 Day 1 | None |
| 006 | Heaviness in right arm | Mild | Possible | 09:20 Day 1 | 09:48 Day 1 | 18:08 Day 1 | None |
| 006 | Erythema around patch | Mild | Possible | 09:20 Day 1 | 10:10 Day 1 | 11:08 Day 1 | None |
| 006 | Erythema at patch site | Mild | Possible | 09:20 Day 1 | 17:20 Day 1 | 19:18 Day 1 | None |
| 006 | Erythema at dressing site | Mild | Remote | 09:20 Day 1 | 16:15 Day 1 | 18:13 Day 1 | None |
| 007 | Erythema at patch site | Mild | Possible | 09:30 Day 1 | 17:30 Day 2 | Ongoing | None |
| 007 | Itchiness at patch site | Mild | Possible | 09:30 Day 1 | 08:00 Day 1 | 18:00 Day 3 | None |
| 008 | Pruritis under patch | Mild | Possible | 09:40 Day 1 | 09:49 Day 1 | 10:30 Day 1 | None |
| 008 | Burning feeling under patch | Mild | Possible | 09:40 Day 1 | 10:23 Day 1 | 12:30 Day 1 | None |
| 008 | Erythema around patch | Mild | Possible | 09:40 Day 1 | 11:10 Day 1 | 12:45 Day 1 | None |
| 008 | Pulling sensation at patch site | Mild | Remote | 09:40 Day 1 | 12:40 Day 1 | 19:34 Day 1 | None |
| 008 | Erythema at patch site | Mild | Possible | 09:40 Day 1 | 17:40 Day 1 | Ongoing | None |
| 008 | Burning sensation at patch site | Mild | Possible | 09:40 Day 1 | 17:42 Day 1 | 18:35 Day 1 | None |
| 008 | Tenderness at patch site | Mild | Possible | 09:40 Day 1 | 18:35 Day 1 | 09:00 Day 3 | None |
| 008 | Headache | Mild | Possible | 09:40 Day 1 | 18:55 Day 1 | 03:20 Day 1 | None |
| 008 | Pruritis at patch site | Mild | Possible | 09:40 Day 1 | 08:30 Day 2 | Ongoing | None |
| 008 | Dry skin at patch site | Mild | Possible | 09:40 Day 1 | 17:41 Day 1 | Ongoing | None |
| 009 | Burning sensation at patch site | Mild | Possible | 09:50 Day 1 | 09:55 Day 1 | 12:47 Day 1 | None |
| 009 | Warm sensation at patch site | Mild | Possible | 09:50 Day 1 | 09:55 Day 1 | 12:47 Day 3 | None |
| 009 | Erythema at patch site | Mild | Possible | 09:50 Day 1 | 17:50 Day 1 | Ongoing | None |
| 009 | Warm sensation at patch site | Mild | Possible | 09:50 Day 1 | 17:50 Day 2 | 10:00 Day 2 | None |
| 009 | Tenderness at patch site | Mild | Possible | 09:50 Day 1 | 17:50 Day 1 | 09:54 Day 3 | None |
| 010 | Erythema around patch | Mild | Possible | 10:00 Day 1 | 10:55 Day 1 | 12:57 Day 1 | None |
| 010 | Warm sensation at patch site | Mild | Possible | 10:00 Day 1 | 10:55 Day 1 | 18:50 Day 1 | None |
| 010 | Tenderness at patch site | Mild | Possible | 10:00 Day 1 | 13:30 Day 1 | 12:00 Day 2 | None |
| 010 | Erythema at patch site | Mild | Possible | 10:00 Day 1 | 18:00 Day 1 | Ongoing | None |
| 010 | Blistering at patch site | Mild | Possible | 10:00 Day 1 | 18:00 Day 1 | 18:00 Day 2 | None |
| 010 | Lethargy | Mild | Remote | 10:00 Day 1 | 08:00 Day 2 | 11:30 Day 2 | None |

The cancer may be a carcinoma, sarcoma, lymphoma, melanoma any malignant cellular tumour, Karposi Sarcoma, marrow bone cancer, Hodgkin's lymphoma, non-Hodgkin's lymphoma and any other viral cancers.

The substance or composition may include at least one physiologically acceptable excipient or carrier. The excipient or carrier may be silicon dioxide e.g. colloidal silicon dioxide.

According to another aspect of the invention, there is provided a method of making a medicament or preparation for the treatment of cancer, rheumatoid arthritis, wasting syndrome diseases, immunomodulatory related diseases, sugar diabetes mellitus 1 or 2 or hepatitis A, B or C the method including the step of combining an active therapeutic agent selected from the group which includes amides of the general formula R₃-CO-NR₁,R₂ in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁, and R₂ are together selected from (CH₂)n, wherein n = 4 or 5, or (CH₂)₂O(CH₂)₂;
R₃ is selected from H, Me and saturated and unsaturated C₂-C₃ alkyl groups;
dialkylsulphoxides of the general formula R₄R₅SO, and mixtures thereof
   with at least one physiologically acceptable excipient or carrier.

R₁, R₂, R₃, R₄ and R₅ may be as hereinbefore described.

The amide may be as hereinbefore described. The dialkylsulphoxide may be dimethylsulphoxide.

The invention extends further to the use, in the treatment of cancer, rheumatoid arthritis, wasting syndrome diseases, immunomodulatory related diseases, sugar diabetes mellitus 1 or 2 or hepatitis A, B or C of a medicament or preparation comprising an active therapeutic agent selected from the group which includes amides of the general formula R₃-CO-NR₁R₂, in which R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or (CH₂)₂O(CH₂)₂;
R₃ is selected from H, Me and saturated and unsaturated C₂-C₃ alkyl groups;
dialkylsulphoxides of the general formula R₄R₅SO, and mixtures thereof.

R₁, R₂, R₃, R₄ and R₅ may be as hereinbefore described.

The medicament or preparation may include at least one physiologically acceptable excipient or carrier.

The physiologically acceptable excipient or carrier may be silicon dioxide e.g. colloidal silicon dioxide.

The amide may be as hereinbefore described. The dialkylsulphoxide may be as hereinbefore described.

The invention extends, further, to the use of a substance or composition in the manufacture of a medicament or preparation for the treatment of cancer, rheumatoid arthritis, wasting syndrome diseases, immunomodulatory related diseases, sugar diabetes mellitus 1 or 2 or hepatitis A, B or C, the substance or composition comprising an active therapeutic agent selected from the group which includes amides of the general formula R₃-CO-NR₁R₂, in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or (CH₂)₂O(CH₂)₂;
R₃ is selected from H, Me and saturated and unsaturated C₂-C₃ alkyl groups;
dialkylsulphoxides of the general formula R₄R₅SO, and mixtures thereof.

R₁, R₂, R₃, R₄ and R₅ may be as hereinbefore described.

The amide may be as hereinbefore described. The dialkylsulphoxide may be as hereinbefore described.

The invention extends, still further, to a method of treating cancer, rheumatoid arthritis, wasting syndrome diseases, immunomodulatory related diseases, sugar diabetes mellitus 1 or 2 or hepatitis A, B or C the method including the step of administering to a subject having cancer, a medicament or preparation comprising a physiologically effective dosage of an active therapeutic agent selected from the group which includes amides of the general formula R₃-CO-NR₁R₂, in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or (CH₂)₂O(CH₂)₂; and
R₃ is selected from H, Me and saturated and unsaturated C₂-C₃ alkyl groups;
dialkylsulphoxides of the general formula R₄R₅SO, and mixtures thereof.

The medicament or preparation may include at least one physiologically acceptable excipient or carrier as hereinbefore described.

R₁, R₂, R₃, R₄ and R₅ may be as hereinbefore described.

The amide may be as hereinbefore described. The dialkylsulphoxide may be as hereinbefore described.

The dosage may be selected so as to provide a blood concentration of the active therapeutic agent, in the blood of the subject being treated, of about 2 - 200 ppm, preferably about 50 - 100 ppm.

The dosage may be administered transcutaneously, i.e. by means of skin transfer of the active therapeutic agent, eg through the application of a skin patch or a cotton wool pad which has been impregnated with the active therapeutic agent or with a composition comprising the active therapeutic agent. The concentration of the active therapeutic agent in the composition used to impregnate the skin patch or cotton wool pad may be about 10 - 100%.

The dosage may be administered about once per week for about 8 hours at a time.

The dosage may, instead, be administered by a method selected from oral dosages, inhalation, by means of a suppository, intravenously and combinations of any two or more thereof. The intravenous introduction will typically be at a controlled rate over a period of time, for example, 1 - 6 gm, more preferably about 4 gm of DMF (99,8% purity) via a saline drip over a period of 6 - 8 hours.

In the case of severe cancers the dosage may be about 14 g at a time e.g. by using two patches each containing about 7g of DMF, typically about once per week, for periods of 6 - 8 hours, preferably about 8 hours.

The invention thus extends to a dosage form for the treatment of cancer which comprises topical application means, such as a skin patch or pad, on which is adsorbed or absorbed an effective amount of an active therapeutic agent as hereinbefore described.

The topical application means may comprise a body of a synthetic polymeric material such as TEFLON (trade name) on which the active therapeutic agent is adsorbed or absorbed. The active therapeutic agent e.g. the dimethylformamide may be present in an amount of about 5 - 15g per patch, preferably about 7g e.g. about 7,064 g. Typically, the dosage form will include DMF admixed with silicon dioxide e.g. colloidal silicon dioxide to form a gel with which the patch is impregnated. The patch may be a Virodene P058 patch.

The invention is now described, by way of example, with reference to the accompanying Figures and Examples, in which
Figure 1 shows plasma DMF concentrations as a function of time during a first set of human toxicity trials;
Figure 2 shows plasma DMF concentrations as a function of time during a second set of human toxicity trials;

### TREATMENT OF CANCER

### EXAMPLE 1

A patient suffering from cancer was treated with dimethylformamide (virodene P058) by the application of skin patches impregnated with a dimethylformamide gel to the patient's body.

The patient was an approximately forty year old woman suffering from metastasised fourth stage breast cancer. The primary cancer was in the left breast and had spread to the right breast. The patient had auxiliary left and right swollen lymph glands under both arms, swelling of lymph glands under the chin and a secondary tumour near the crown of the head protruding from the skull which was four fingertips across. The patient was dehydrated and unable to keep down food or water. The primary tumour in the left breast had a circumference of 52 cm and a diameter of 32 cm.

A skin patch was applied to the skin adjacent the tumour for six hours. The skin patch contained about 7,064g of a gel comprising DMF (92,5 % m/m) and colloidal silicon dioxide (7,5 % m/m). The gel served to prevent leakage of liquid DMF from the patches. The patches were manufactured at most 12 hours prior to use as DMF evaporates rapidly.

Two days later the tumour circumference had reduced to 51 cm and diameter to 29,5 cm and the patient was able to consume fruit and vegetable juices. The treatment was continued in the same fashion on alternative days for a further week followed by a break of one week. Three days after the treatment began the patient's appetite returned and the patient was able to hold down food. The area of the tumour closest to the patches appeared to be shrivelled and contracted and the tumour reduced in size by about 1 - 2 mm per week. There was notable protraction of the blood vessels and tumour support system. The temperature of the tumour also dropped to normal.

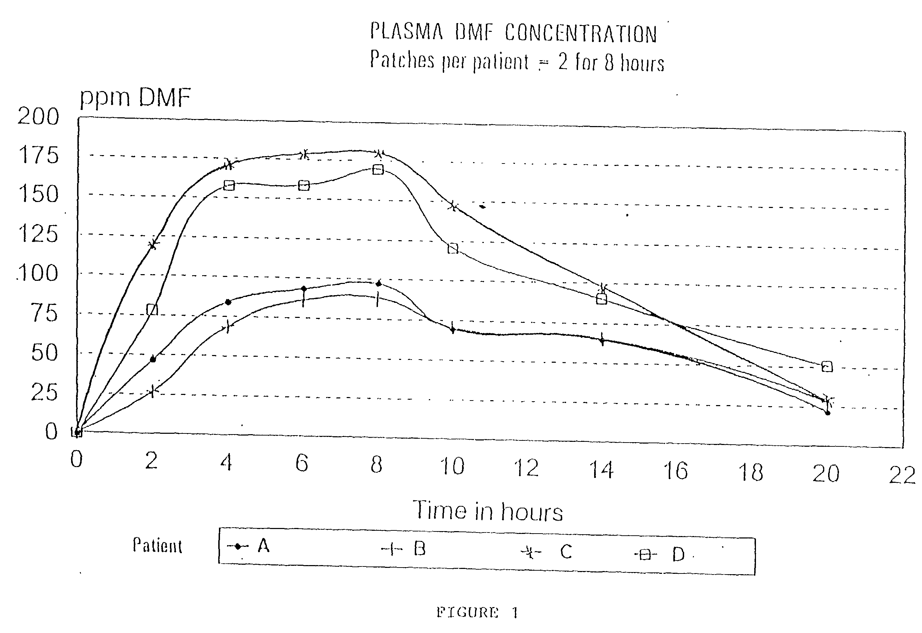

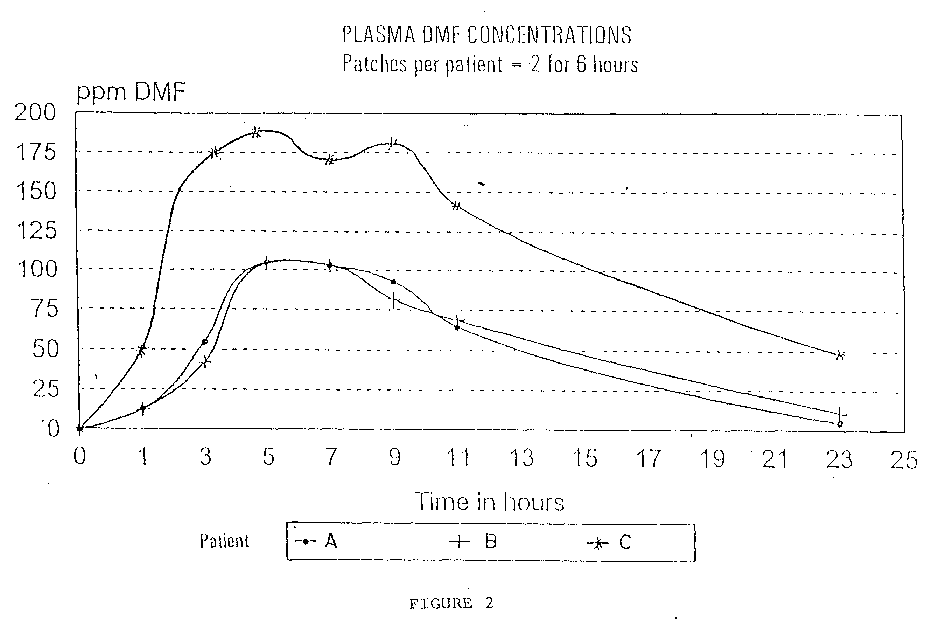

The intended level of DMF in the patient's blood is 100 --300 ppm. For a patient weighing about 60 kg, an amount of about 14 g over a period of 12 hours is required to produce a level of 100 ppm. To obtain a blood level of 100 ppm about 1,272 g of DMF must be absorbed per hour, thus each patch requires about 7,064 g DMF to deliver the required amount, having a surface area of 6,36 cm² (each patch). DMF absorption rate is about 9.4 mg/cm²/hour. In theory this treatment will deliver 125 - 135 ppm, but due to evaporation of the DMF, 100 ppm is obtained. Absorption capability varies from patient to patient depending on factors such as skin-type and skin thickness. To obtain the desired levels of DMF in a patient, plasma DMF concentrations were monitored for the patient and treatment adjusted accordingly depending on the DMF level of the patient (see Figures 1 and 2 for examples of various plasma DMF concentration levels in patients receiving the same treatment).

The stickers or patches were thus each loaded with about 7,064 g of the gel of DMF and silicon dioxide. Each patch was applied for a period of 6 hours, on alternate days for one week followed by a one week break.

Prior to the treatment with dimethylformamide, a comprehensive base-line clinical and psychological evaluation of the patient was conducted. The evaluation provided base-line biochemical and haematological data on the patient.

The concentration of DMF in the patient's blood was determined hourly during the period of treatment. An intravenous line was introduced each morning to take blood samples and was kept open with an infusion of Normal Saline at a rate of 20mℓh and daily monitoring of the active metabolise AMCC (eg by 4 hourly urine sampling) derived from the DMF was also conducted. Subsequent applications of DMF were adjusted in accordance with measured changes in blood level DMF concentration resulting from changes in absorbtion variables and daily full haematological and biochemical profiles were conducted to detect any changes in liver function. Daily full clinical and psychological evaluations were also conducted.

All clinical and laboratory data was fed into a centralised data system to facilitate rapid response to any detrimental change so as to curtail treatment to maximise clinical effect and minimise potential side effects.

The tests included
a) Serum:- S-Na, S-K, S-Cl, S-CO₂,S-Urea, Surate, S-Creat, SCa, S-Ca, S-Mg, S-Phos, Serum Total, S-Conjd;
b) Protein Electrophoresis: ST-Protein, S-Albumin, S-Total Glob, S-Alphal Glob, S-Alpha2 Glob, S-Beta Glob, S-Gamma Glob;
c) White cell differential count:-Wh cell count, Neutro absolute, Lypho absolute, Mono absolute, Eosino absolute, Baso absolute;
d) Liver Enzymes: - S-Alk. Phos, S-Gamma GT, S-Alt (SGPT), S-AST(SGOT), S-LD;
e) blood analysis for DMF levels;
f) urine analysis for AMCC levels

### EXAMPLE 2

A second female patient suffering from Mama Carcinoma was terminally ill and had a tumour with a diameter of 53 cm on her left breast. After three weekly treatments using skin patches impregnated with 7.04g of dimethylformamide for 4 hours over a period of 3 weeks, the tumour had shrunk by 7 cm in diameter. The patient experienced less pain and bleeding disappeared.

### EXAMPLE 3

A third patient was a male suffering from full blown AIDS. The patient had seven tumours which were diagnosed as Karposi Sarcoma. After two, weekly treatments with skin patches impregnated with 7.04g of dimethylformamide, each applied for 8 hours once a week for two weeks, the tumours faded. After six treatments with the same amount of dimethylformamide the tumours disappeared and after 18 months no tumours had recurred.

### EXAMPLE 4

A fourth patient was an adult female diagnosed with non-Hodgkins Lymphoma. The patient had not been on chemotherapy and was HIV positive. After eight treatments using skin patches impregnated with 7.04g dimethylformamide for 8 hours at a time over a period of 8 weeks, the cancer disappeared. Eight months later the cancer had not recurred.

In further studies, it was found that *in-vitro* studies in different cell cultures expressed properties of benign better-differentiated phenotypes when these cultures were exposed to polar substances. DMF caused conformational changes and hypomethylation of DNA sequences, and inhibited expression of myc onco-proteins. A marked alteration in the random piling, non-contact-inhibited growth pattern of virus transformed mice kidney cell-lines, occurred when the normal growth medium was supplemented by 0.5% DMF concentration. Instead of a piling pattern, mono layers of cells in regular parallel orientation formed that were typical of non-malignant fibroblasts. Similar effects were noted on other cell-lines such as human colon-carcinoma cells. Focal Adhesion Kinase (FAK) is activated in response to receptor activation at focal adhesion sites. FAK is a substrate for the oncogenic forms of the src-family of tyrosine kinases. The action of polar agents on oncogenic expression is believed to be the induction of cancer cells to become more benign. It would seem reasonable that to convert a malignant cell to a benign phenotype, there should be some modulation of gene expression causing malignancy in the first place. In the HL-60 human promyelocytic leukemia cells, dimethylsulfoxide (DMSO) reduced the expression of c-myc oncogenes by 80 to 90%. Some brain tumours are regulated by glial-binding growth factors such as Epidermal Growth Factor (EGF) and Platelet Derived Growth Factor (PDGF) both of which are related to oncogenes. The PDGF β-chain gene is the cellular homologue and proto-oncogene of the viral erb-β oncogene. Cell maturation induced by polar agents (N-MF) as single agents is most likely to be effective in Neuroblastoma and Glioblastoma (as well as leukemia and melanoma). Polar agents easily penetrate the blood brain barrier and sensitize tumour cells to both X-radiation and cis-platinum. There is a proven link between PDGF, Transforming Growth Factor-α (TGF-α), EGF, EGF-receptors and the neoplastic changes causing brain tumours.

### Primary Mode

DMF is a cell differentiator which works on gene expression (DNA) of the tumeric cell. This is a primary mechanism for activating a gene in the binding of an inducer protein to a promoter side of the head of the gene. The gene remains in a turned on state, directing the synthesis of more protein, until something causes it to be switched off. A common mechanism of gene regulation is negative feedback. The presence of a large amount of the protein product from the gene can interfere with the ticking of the inducer that originally turned the gene on. When this happens, the gene is turned off. This thermostat-like mechanism turns genes on and off, maintaining a supply of product balance against consumption. When a specific external signal molecule binds to the cell receptor, an enzyme called protein kinase located on the internal side of the cell membrane is activated, this specific protein helps with the increase of forming tumor cells. It is known that DMF inhibits this specific protein which renders the tumor cell into a latent state.

The differential effects of DMF in transforming growth factor-beta 1 on human ovarian cancer cell line. (HOC-7) to a malignant cell. This was also found in human colon carcinoma. (Factor - beta 1 is growth factor, that can be up regulated or transformed in human cells.)

Exposure of some tumor cells to regulators of cell differentiation causes induced dependent alterations of the antigenic pattern of the cell.

Exposure of HOC-7 ovarian adenocarcinoma cells to regulators of cell differentiation caused inducer-dependent alterations of the antigenic pattern of the cells. Immunocytochemistry revealed that N,N-dimethylformamide (DMF) elevated the membrane staining for epidermal growth factor (EGF) receptor and for desmoplakins I and II. DMF also stimulated cytoplasmic and surface labeling for CA 125 and the disposition of fibronectin into the extracellular matrix. Stimulation of fibronectin was also seen after addition of transforming growth factor (TGF) - beta 1. The immunosorbent assay (ELISA) revealed that DMF dose dependently induced expression of EGF - receptor. CA 125, Fibronectin, and desmoplakins I and II. TGF - beta 1 stimulated fibronectin and desmoplakins I and II only.

Production of EGF and TGF - alpha was not affected by these inducers. Immunocytochemistry. ELISA and Western blotting showed that both inducers cause down-regulation of myconcoproteins. DMF was more effective in changing the immunophenotype of HOC-7 cells than TGF-beta 1. Demoplkins I and II demonstrated elevated epithelial differentiation, whereas fibronectin indicated stimulation of extracellular matrix formation. Elevated EGF-receptor could not compensate for the growth inhibition induced by DMF. The expression of myconcoproteins was inversely related to cell proliferation CA 125, however, seems to be unrelated to cell growth.

Treatment of chemistry transformed fibroblasts with N,N-dimethylformamide (DMF) results in the restoration of a nontransformed phenotype. In an attempt to identify more precisely the mechanisms by which DMF reverses the transformed phenotype, the effects of DMF on fibroblasts which were transformed by a single gene - specifically a synthetic epidermal growth factor (EGF) gene or the Harasoncogene were examined. The constitutive expression of either the Ha-rasrasoncogene or the EGF gene in FR3T3 fibroblasts resulted in cellular transformation. The effect of the differentiation - inducing agent DMF on several properties of these transformed cell lines was examined.

The EGF-transfected cells resulted in the inhibition of anchorage-independent growth, the restoration of a normal cellular morphology and growth rate in monolayer culture, and the down-regulation of the proliferation-associated nucleolar protein B23. DMF treatment has a much slighter effect on growth of the ras-transfected cells in monolayer culture or under anchorage-independent growth conditions.

The high proliferation rate of the ras-3 cells was associated with elevated expression of protein B23. The 19-3 cells. But not the ras-3 cells, expressed cell surface fibronectin. Treatment of the ras-3 cells of DMF did not restore fibronectin expression. The binding of EGF was increased in rastransfected cells, but in neither case did DMF alter EGF binding. DMF treatment increased the secretion of EGF in the 2 transfected lines as well as in control cells. These results suggest that the aberrant-growth control in the EGF-transfected cells, but not in the ras-transfected cells, could be modulated by DMF and that the aberrant-growth control mechanisms were different in these 2 cell types.

### Secondary Mode

Tyrosine Kinase acts in the signalling pathway at cellular level with growth factor receptors, tumor necrosis factor-α, interleukin-6 and interleukin-12. Growth factors send signals to the cell nucleus via a chain of interacting proteins. Several proteins in this cascade are the product of cytoplasmic oncogenes. Most growth-factor receptors with (PTK) Protein Tyrosine Kinase activity dimerize in response to ligand binding. Their catalytic activity is increased, causing transphosphorylation of a receptor pair. These phosphorylated tyrosine residues create binding sites for proteins containing a sequence called an SH2 (src-homology region 2) domain. SH2 domains recognize, in addition to phosphorylated tyrosine a short surrounding linear sequence, often of about four amino-acids, providing their specificity of interaction. On binding to the receptor, the enzyme may become phosphorylated on tyrosine. In some cases interaction itself seems to be the activating factor, perhaps by inducing a conformational change. The signal is then passed on to downstream molecules, with consequent effects on the nuclear DNA.

Mutations have been found in ras genes in many human tumor types, with a high prevalence. At various levels in the signal-transduction pathway, individual proteins have been found to be altered by mutations in human cancers. The aberrant activation of the information-cascade at many levels can result in the same end-point namely excessive growth stimulation.

Growth factor receptors span the plasmid membrane such that binding of extra-cellular factor elicits biochemical changes within cells that are required for cell proliferation. A large number of oncogenes are derived from genes that encode growth factor receptors. Many were initially discovered associated with oncogenic retro-viruses and function enzymatically as Protein Tyrosine Kinases (PTK). The fact that many growth factor receptors and oncogenes are tyrosine kinases is significant because phospho-tyrosine normally constitutes only a small fraction (0.1%) of the total protein phosphate. This suggests that tyrosine phosphorylation is frequently reserved for pathways controlling cell growth and differentiation. A number of mitogenic factors bind to membrane spanning glyco-proteins belonging to the growth hormone receptor super-family. These include Interleukins 2,4,6 and 7 and haemopoetic colony stimulating factors (G-CSF, GM-CSF, IL-3 and erythropoietin). Although there is no tyrosine kinase domain for the haemopoietic cyrokine receptors, it is known that stimulation of these receptors leads to an increase in cytoplasmic tyrosine kinase activity. Several new enzymes were found and two of these, JAK1 and JAK2, appeared to have multiple kinase domains. It now appears that JAK1 and JAK2 associate with many growth factor / cytokine receptor systems (e.g. EGFR and G-CSF receptor) and they are activated as a result of ligand binding.

### Ligand regulated Enzymes

The ligand regulated enzymes represent a huge family of receptors that can be divided into different families. The unifying structural feature of these receptors is the presence of an extra-cellular ligand-binding domain that regulates activity of an intra-cellular catalytic domain. In most instances the two domains of the receptor are connected by a single trans-membrane spanning region. This super-family includes a variety of growth factors, cytokines and peptides. The structural and functional properties of these receptors have been reviewed and found to have an intra-cellular tyrosine domain in common.

One of the major groups within the ligand-regulated enzyme super-family is the receptor-tyrosine kinase family which includes receptors for Fibroblast Growth Factor (FGF), EGF and PDGF.

The next group that includes receptors for insulin and insulin-like growth factor are heterotetrameric consisting of two α and two β sub-units connected by disulfide bonds. The two α sub-units contribute to the ligand binding domain, whereas the two β sub-units traverse the membrane and possess tyrosine kinase activity.

The class which includes the FGF, has three immunoglobulin-like domains in the extra cellular ligand binding portion of the receptor.

Another member of the ligand-regulated enzyme super family is the receptor for Atrial Natriuretic Peptide (ANP).

The last group within the ligand-regulated enzyme super family is a family that includes receptors for NGF and TNF. The intra-cellular domain has an unknown function and it lacks a sequence identity with any other known proteins. It has been suggested that a trans-membrane tyrosine kinase can interact with the intra-cellular domain of the NGF receptor to elicit a biological signal. Once occupied by their respective ligands, the NGF causes neuronal differentiation and survival whereas the TNF receptor causes inflammation and tumor cell death.

Considering the above analysis the Applicant believes that the group of TPK (protein tyrosine kinases) play a central role in cancer. It is a fact that PTK is a commonly occurring factor as second messenger in the pathogeneses of these disease states and it is assumed that modifying this enzyme action with polar substances, the disease state's progression could be altered.

Growth factors play an important role in inducing and promoting neoplastic disease. Neoplastic cells as such, can produce more growth factors. The group of protein tyrosine kinases is an important link in the pathway from receptor to nucleus as a second messenger. DMF inhibits this pathway and alters the growth pattern as well as the multiplication of tumor cells. The correct application, and the correct duration of application, has been found to prevent the growth and metastasizing effects of neoplastic disease.

In-vitro studies were carried out to show the effect of DMF on PTK. A Tyrosine Kinase Assay kit (non-radioactive # 1534505, Boehringer Mannheim) was used to show that DMF is a Tyrosine Kinase inhibitor. The results are set out in the Appendix below.

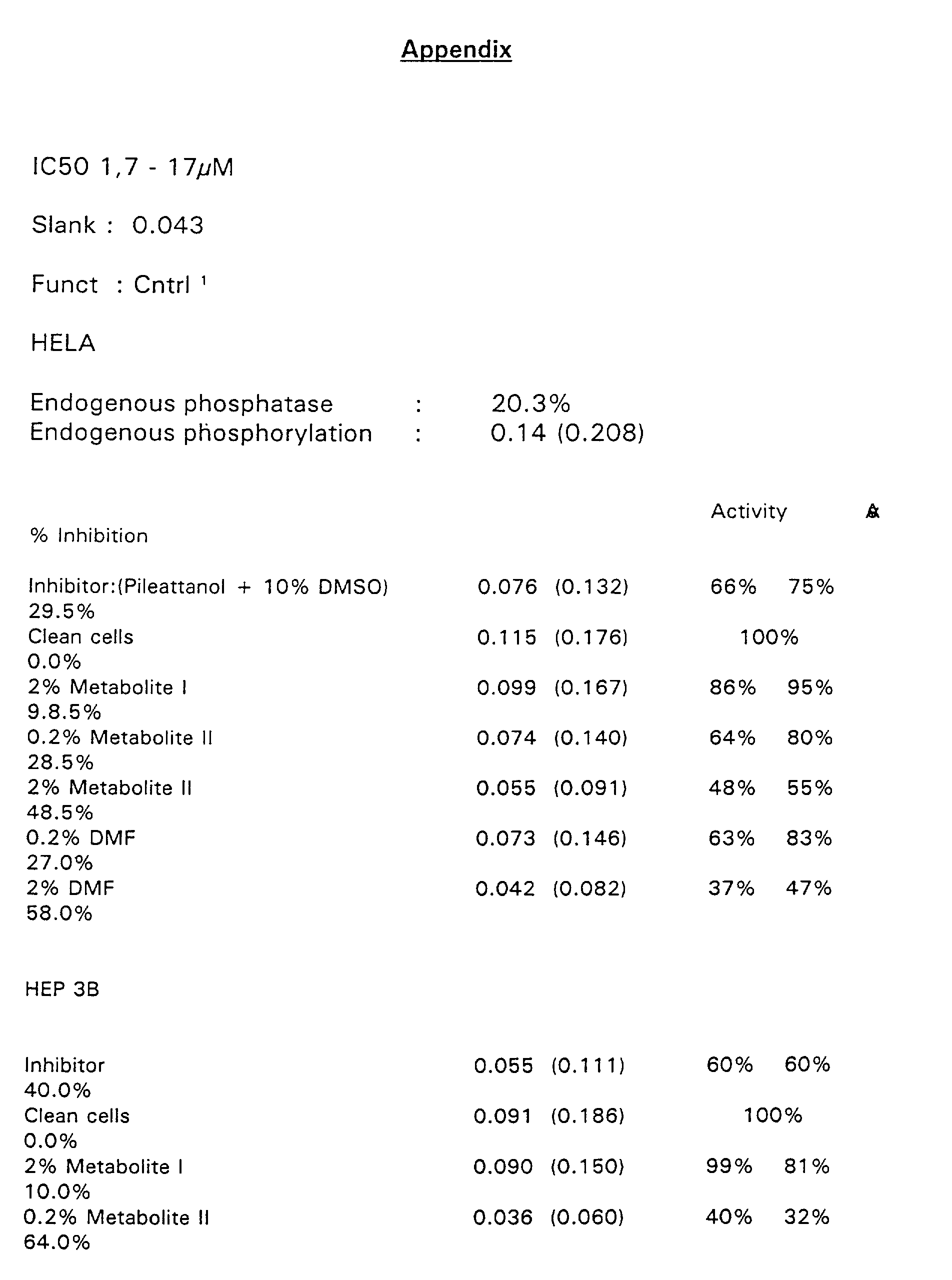

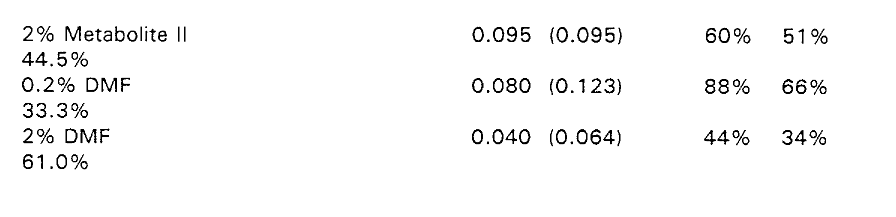

It is an advantage of the invention illustrated that the toxicity of DMF is about 5 - 10 times lower when administered subcutaneously as compared with oral administration. The Applicant believes that DMF affects or destroys certain enzymes associated with cancer and causes dehydration of the tumours.

## Claims

1. A substance or composition for the treatment of cancer, rheumatoid arthritis, wasting syndrome diseases, immunomodulatory related diseases, sugar diabetes mellitus 1 or 2 or hepatitis A, B or C, the substance or composition comprising an active agent selected from the group consisting of amides of the general formula R₃-CO-NR₁,R₂, in which:
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or are (CH₂)₂O (CH₂)₂; and
R₃ is selected from H, Me and saturated and unsaturated C₂ - C₃ alkyl groups;
dialkylsulphoxides of the general formula R₄R₅SO, and mixtures thereof.

2. A substance or composition as claimed in claim 1, in which at least one of R₁ and R₂ is a methyl group.

3. A substance or composition as claimed in claim 1 or claim 2, in which at least one of R₄ and R₅ is a methyl group.

4. A substance or composition as claimed in any one of the preceding claims, in which at least one of R₁, R₂, R₄ and R₅ is a fluorinated C₁-C₃ alkyl group.

5. A substance or composition as claimed in Claim 1, in which the active agent is selected from the group including formamide, methyl formamide, dimethylformamide, acetamide, methylacetamide, dimethylacetamide, diethylacetamide, isopropylacetamide, diisopropylacetamide, N-acetylpiperidine, N(β-hydroxyethyl) acetamide, N,N-di(β-hydroxyethyl) acetamide, N-acetylmorpholine, acrylamide, propionamide, N-fluoromethyl-N-methyl-formamide, dimethylsulphoxide and mixtures of any two or more thereof.

6. A substance or composition as claimed in claim 5, in which the active agent is dimethylformamide C₃H₇NO (DMF).

7. A substance or composition as claimed in claim 5, in which the active agent is *N*-fluoromethyl-*N*-methylformamide.

8. A substance or composition as claimed in claim 5, in which the active agent is dimethylsulphoxide (DMSO).

9. A substance or composition as claimed in any one of the preceding claims, in which the cancer is selected from a carcinoma, sarcoma, lymphoma, melanoma, malignant cellular tumour, Karpo 21 Sarcoma, marrow bone cancer, Hodgkin's lymphoma, non-Hodgkin's lymphoma and viral cancers.

10. A substance or composition as claimed in any one of the preceding claims, which includes at least one physiologically acceptable excipient or carrier.

11. A substance or composition as claimed in claim 10, in which the excipient or carrier is silicon dioxide.

12. A method of making a medicament or preparation for the treatment of cancer, rheumatoid arthritis, wasting syndrome diseases, immunomodulatory related diseases, sugar diabetes mellitus 1 or 2 or hepatitis A, B or C, the method including the step of combining an active therapeutic agent selected from the group which includes amides of the general formula R₃-CO-NR₁,R₂ in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁, and R₂ are together selected from (CH₂)n, wherein n = 4 or 5, or are together (CH₂)₂O(CH₂)₂;
R₃ is selected from H, Me and saturated and unsaturated C₂-C₃ alkyl groups;
dialkylsulphoxides of the general formula R₄R₅SO and mixtures thereof
with at least one physiologically acceptable excipient or carrier.

13. A method as claimed in claim 12, in which at least one of R₁ and R₂ is a methyl group.

14. A method as claimed in claim 12 or claim 13, in which at least one of R₄ and R₅ is a methyl group.

15. A method as claimed in any one of claims 12 to 14, in which at least one of R₁, R₂, R₄ and R₅ is a fluorinated C₁-C₃ alkyl group.

16. A method as claimed in Claims 12, in which the active agent is selected from the group including formamide, methyl formamide, dimethylformamide, acetamide, methylacetamide, dimethylacetamide, diethylacetamide, isopropylacetamide, diisopropylacetamide, N-acetylpiperidine, N(β-hydroxyethyl) acetamide, N,N-di(β-hydroxyethyl) acetamide, N-acetylmorpholine, acrylamide, propionamide, N-fluoromethyl-N-methyl-formamide, dimethylsulphoxide and mixtures of any two or more thereof.

17. A method as claimed in claim 16, in which the active agent is dimethylformamide C₃H₇NO (DMF).

18. A method as claimed in claim 16, in which the active agent is *N*-fluoromethyl-*N*-methylformamide.

19. A method as claimed in claim 16, in which the active agent is dimethylsulphoxide (DMSO).

20. Use, in the treatment of cancer, rheumatoid arthritis, wasting syndrome diseases, immunomodulatory related diseases, sugar diabetes mellitus 1 or 2 or hepatitis A, B or C, of a medicament or preparation comprising an active therapeutic agent selected from the group which includes amides of the general formula R₃-CO-NR₁ R₂, in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or are together (CH₂)₂O(CH₂)₂;
R₃ is selected from H, Me and saturated and unsaturated C₂-C₃ alkyl groups; dialkylsulphoxides of the general formula R₄R₅SO, and mixtures thereof.

21. Use as claimed in claim 20, in which the medicament or preparation includes at least one physiologically acceptable excipient or carrier.

22. Use as claimed in claim 21, in which the physiologically acceptable excipient or carrier is silicon dioxide.

23. Use as claimed in any one of claims 20 to 22 inclusive, in which at least one of R₁ and R₂ is a methyl group.

24. Use as claimed in any one of claims 20 to 23 inclusive, in which at least one of R₄ and R₅ is a methyl group.

25. Use as claimed in any one of the preceding claims 20 to 24 inclusive, in which at least one of R₁, R₂, R₄ and R₅ is a fluorinated C₁-C₃ alkyl group.

26. Use as claimed in Claim 20, in which the active agent is selected from the group including formamide, methyl formamide, dimethylformamide, acetamide, methylacetamide, dimethylacetamide, diethylacetamide, isopropylacetamide, diisopropylacetamide, N-acetylpiperidine, N(β-hydroxyethyl) acetamide, N,N-di(β-hydroxyethyl) acetamide, N-acetylmorpholine, acrylamide, propionamide, N-fluoromethyl-N-methyl-formamide, dimethylsulphoxide and mixtures of any two or more thereof.

27. Use as claimed in claim 26, in which the active agent is dimethylformamide C₃H₇NO (DMF).

28. Use as claimed in claim 26, in which the active agent is *N*-fluoromethyl-*N*-methylformamide.

29. Use as claimed in claim 26, in which the active agent is dimethylsulphoxide (DMSO).

30. Use of a substance or composition in the manufacture of a medicament or preparation for the treatment of cancer, rheumatoid arthritis, wasting syndrome diseases, immunomodulatory related diseases, sugar diabetes mellitus 1 or 2 or hepatitis A, B or C, the substance or composition comprising an active therapeutic agent selected from the group which includes amides of the general formula R₃-CO-NR₁R₂, in which
R₁ and R₂ are independently selected from the group including H, Me, halomethyl, saturated and unsaturated C₂-C₃ alkyl groups, saturated and unsaturated halogenated C₂-C₃ alkyl groups, hydroxylated alkyl groups; or
R₁ and R₂ are together selected from (CH₂)n, wherein n = 4 or 5, or are together (CH₂)₂O(CH₂)₂;
R₃ is selected from H, Me and saturated and unsaturated C₂-C₃ alkyl groups; dialkylsulphoxides of the general formula R₄R₅SO, and mixtures thereof.

31. Use as claimed in claim 30, in which at least one of R₁ and R₂ is a methyl group.

32. Use as claimed in claim 30 or claim 31, in which at least one of R₄ and R₅ is a methyl group.

33. Use as claimed in any one of the preceding claims 30 to 32 inclusive, in which at least one of R₁, R₂, R₄ and R₅ is a fluorinated C₁-C₃ alkyl group.

34. Use as claimed in Claim 30, in which the active agent is selected from the group including formamide, methyl formamide, dimethylformamide, acetamide, methylacetamide, dimethylacetamide, diethylacetamide, isopropylacetamide, diisopropylacetamide, N-acetylpiperidine, N(β-hydroxyethyl) acetamide, N,N-di(β-hydroxyethyl) acetamide, N-acetylmorpholine, acrylamide, propionamide, N-fluoromethyl-N-methyl-formamide, dimethylsulphoxide and mixtures of any two or more thereof.

35. Use as claimed in claim 34, in which the active agent is dimethylformamide C₃H₇NO (DMF).

36. Use as claimed in claim 34, in which the active agent is *N*-fluoromethyl-*N*-methylformamide.

37. Use as claimed in claim 34, in which the active agent is dimethylsulphoxide (DMSO).

38. A dosage form for the treatment of cancer, rheumatoid arthritis, wasting syndrome diseases, immunomodulatory related diseases, sugar diabetes mellitus 1 or 2 or hepatitis A, B or C,which comprises topical application means on which is adsorbed or absorbed an effective amount of an active therapeutic agent which includes a substance or composition as claimed in any one of claims 1 to 11 inclusive.

39. A dosage form as claimed in claim 38, in which the topical application means comprises a body of a synthetic polymeric material on which the active therapeutic agent is adsorbed or absorbed.

40. A dosage form as claimed in claim 39, which includes DMF admixed with silicon dioxide to form a gel with which the patch is impregnated.

41. A subtance or composition as claimed in Claim 1, sustantially as herein described and illustrated.

42. A new substance or composition substantially as herein described.

43. A new method of making a medicament as claimed in Claim 12, substantially as herein described and illustrated.

44. A new method of making a medicament substantially as herein described.

45. Use of a medicament or preparation as claimed in Claim 20, substantially as herein described and illustrated.

46. A new use of a medicament or preparation substantially as herein described.

47. Use of a substance or composition in the manufacture of a medicament as claimed in Claim 30, substantially as herein described and illustrated.

48. A new use of a substance or composition in the manufacture of a medicament substantially as herein described.

49. A dosage form for the treatment of cancer as claimed in Claim 38, substantially as herein described and illustrated.

50. A new dosage form for the treatment of cancer substantially as herein described and illustrated.
